(19) Europäisches Patentamt

European Patent Office

Office européen des brevets

(11) **EP 1 707 219 A2**

(12) **EUROPEAN PATENT APPLICATION**

(43) Date of publication:
**04.10.2006 Bulletin 2006/40**

(51) Int Cl.:
***A61K 51/04*** *(2006.01)* ***A61K 51/02*** *(2006.01)*
***A61K 51/12*** *(2006.01)*

(21) Application number: **05110496.6**

(22) Date of filing: **08.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.04.2005 JP 2005001700**

(71) Applicant: **Atomic Energy Council - Institute of Nuclear**
**Energy Research**
**Lungtan, Taoyuan,**
**Taiwan (TW)**

(72) Inventors:
• **Lee, Shui-Cheng**
**333, CHIAAN VILLAGE, LUNGTAN, TAOYUAN (CN)**

• **Wang, Mei-Hui**
**333, CHIAAN VILLAGE, LUNGTAN, TAOYUAN (CN)**
• **Chiang, Tung-Chian**
**333, CHIAAN VILLAGE, LUNGTAN, TAOYUAN (CN)**
• **Farn, Shiou-Shiow**
**333, CHIAAN VILLAGE, LUNGTAN, TAOYUAN (CN)**

(74) Representative: **Beck, Michael Rudolf et al**
**Beck & Rössig**
**European Patent Attorneys**
**Cuvilliésstrasse 14**
**81679 München (DE)**

(54) **Radioactive kits for a gastric emptying measurement**

(57) The present invention provides a test meal kits that arc used in the diagnosis of gastrointestinal disorders characterized by changes in the rate of gastric emptying; and, with a breath test or a nuclear scintigraphy scan, arc used to measure a half-gastric emptying time useful for therapy monitoring of gastrointestinal disorders in clinical.

FIG.3

EP 1 707 219 A2

**Description**

FIELD OF THE INVENTION

The present invention relates to a measurement; more particularly, relates to a test meal kits that are used in the diagnosis of gastrointestinal disorders characterized by changes in the rate of gastric emptying, which kit contains an isotope tracer and a dry mix provided separately to minimize concerns on the stability and the FDA regulations; and, with a breath test or nuclear scintigraphy scan, to measure a half-gastric emptying time useful for therapy monitoring of gastrointestinal disorder in clinical.

BACKGROUND OF THE INVENTION

A current method for measuring gastric emptying, called a nuclear scintigraphy scan, uses a radioactive material of a Tc-99m (metastable Technetium-99) sulfur colloid which is injected to an egg to be further prepared as an omelet; and, requires the patient to lie still for more than three hours for a scanning. There are many disadvantages. First, the between-day coefficient variance of the measurement for an individual is more than 20% since the Tc-99m sulfur colloid in the omelet does not distribute homogeneously. Second, although stuffs are fresh-made and fresh-used, the preparation process is inconvenient and difficult to control quantity. Furthermore, it needs expensive nuclear imaging suites, usually available only in major centers, and its cost effect is low. So, the expense and the inconvenience of the scintigraphy test lead to the creation of a simplified breath test.

The breath test for the measurement of a gastric emptying of solids, labeled with a carbon-13 ($^{13}$C) octanoic acid or a carbon-14 ($^{14}$C) octanoic acid, is referred to Ghoos et al (1993), "Measurement of Gastric Emptying Rate of Solids by Means of a Carbon-Labeled Octanoic Acid Breath Test", Gastroenterology 104, 1640-1647 and Maes et al (1994), "Combined Carbon-13-Glycine/Carbon-14-Octanoic Acid Breath Test to Monitor Gastric Emptying Rates of Liquids and Solids", J Nucl Med 35, 824-831. In brief, after an overnight fast, the subject is given a test meal comprising a scrambled egg with the yolk doped with a $^{13}$C octanoic acid or a $^{14}$C octanoic acid. The yolk and the egg white are baked separately but are injected together with two slices of white bread and margarin, followed immediately by water. The test is based on a prompt solubilization of the $^{13}$C octanoic acid or the $^{14}$C octanoic acid in an egg yolk and the disintegration of the labeled solid phase in the duodenum, followed by a rapid absorption by the intestinal cells and a preferential oxidation to $^{13}$CO$_2$ in the liver. The appearance of $^{13}$CO$_2$ in a breath is primarily determined by the rate of delivery of the test meal from the stomach into the duodenum. Breath samples are collected and analyzed to get a half-emptying time and a lag phase, which are parameters for the calculation of a gastric emptying rate. All stuffs are fresh-made and fresh-used. The preparation is time consuming and it is hard to control the quality and the quantity. Besides, the coefficient variance of the measurement is more than 20% and the shelf-life is short. There can be difficulty on a uniform incorporation of the isotope tracers into the egg, since only the yolk mixed with $^{13}$C-octanoic acid or $^{14}$C-octanoic acid yet not the whole egg. In addition, meal homogeneity is difficult to maintain. Eggs, for example, vary in caloric content, size and composition, so that non-standardized cooking conditions can affect the outcome of the test and prevent intra-clinic comparison of the test results. Furthermore, the palatability was less than desirable because of the unpleasant taste, the pungent aroma of the octanoic acid, and the high viscosity at a room temperature.

For solving these problems, Peter (DKUS5707602, 1998), Spathe (Isot. Environ. Health Stud., 1998) and Meiler (WO02/062399A1) provide a biscuit with a sealed storage which is prepared with either $^{13}$C-Spidina platenesis to wheat, or $^{13}$C sodium acetate to wheat or sugar syrup. Pre-made products certainly have a shorter shelf-life than a dry mix. In addition, the incorporation of $^{13}$C isotope tracer directed into the biscuit presents FDA regulatory hurdles that must be addressed, which can be avoided by not mixing the $^{13}$C isotope tracer into a food product. Additionally, the growth of algae under specialized conditions costs additional expenses to the final test. The algae also may cause an adverse allergic reaction to a patient and may be less than palatable. The $^{13}$C sodium acetate is not evenly incorporated into the food so that the CV (coefficient variance) of a gastric emptying measurement with a $^{13}$C sodium acetate breath test is still more than 10%. Furlhermore, the chemical stability of $^{13}$C sodium acelale is poor so that the accuracy of the results is hard to maintain. Ghoos (2001, WO01/72342A1) prepares a test cake through a microwave by instantly mixing a dry egg yolk mix and $^{13}$C octanoic acid. Wagner (2003, US6548043 B1) stores a $^{13}$C octanoic acid and a standardized dry mix separately. The dry mix for the test meal is standardized in several respects, including the caloric content, the volume, the carbohydrate, the fat and the protein proportions; and is packed in a stable dry mix form, which can be easily shipped and stored indefinitely at room temperature. The test meal is constituted on site with liquid and $^{13}$C octanoic acid; then, is cooked and is administered to a patient, followed by an appropriate diagnostic measurement, such as a $^{13}$CO$_2$ breath test. One of the disadvantages is that the delivery system is only allowed to measure the solid emptying due to the insolubility of the $^{13}$C octanoic acid. Other disadvantages include the high cost of the octanoic acid, the low speed of adsorption and metabolism in body, and the longer testing time required. Although the test meal is made by an instant solubilization and an instant preparation and is very close to a true meal either in the caloric content or in the nutrition proportions, inconvenience still exists that it is not palatable and toxic duc to the characteristics of the octanoic acid and so it limits its clinical usage. In addition, the CV of the gastric emptying measurement by a $^{13}C$ octanoic acid breath test is more than 20%. The precision is poor and it could not be applied to a liquid or a semi-solid gastric emptying system due to the water insolubility of the octanoic acid. So, the prior arts do not fulfill users' requests on actual use.

SUMMARY OF THE INVENTION

The present invention is a standard, easy to use, and rapidly absorbed test meal kit, which can be applied to measure a solid or semisolid gastric mobility. Therein, the albumin of egg powder of the kit is coagulated into a solid form with an isotope tracer at more than 75°C and the isotope tracer is well incorporated into the food product.

The present invention also provides a rapid test method with low cost for a gastric emptying measurement, comprising the following steps:

(a) Rapidly constituting a solid test meal, comprising a dry mix, an isotope tracer (such as a $^{13}C$ glycine, a $^{14}C$ glycine, a Tc-99m phytate, a Tc-99m-sulfur colloid, or a Tc-99m DTPA) and water, by mixing the dry mix, the isotope tracer and the water and cooking the test meal in 9 minutes.
(b) Collecting breath samples from the patients before administering the test meal to the patient.
(c) Orally administering the solid meal in 10 minutes. The Isotope tracer is not adsorbed or metabolized in the stomach, since the isotope tracer is well incorporation in test meal and is chemically stable in the gastric juice.
(d) Collecting breath samples from the patients per 15 minutes for four hours after administering the test meal to the patient.
(e) If the isotope tracer is a $^{13}C$ glycine or a $^{14}C$ glycine, measuring the amount of $^{13}CO_2$ or $^{14}CO_2$ by a carbon isotope breath test to determine the gastric half emptying time of the patient. If the isotope tracer is a Tc-99m phytate, a Tc-99m sulfur colloid, or a Tc-99m DTPA, measuring the gamma count around stomach by a gamma-camera to determine the gastric half emptying time of the patient.

In the preferred embodiment, the isotope tracer is labeled with a $^{13}C$ glycine, a Tc-99m phytate, a Tc-99m sulfur colloid, a Tc-99m DTPA or a $^{14}C$ glycine. A $^{13}C$ glycine could be a crystal, a capsule, a tablet, a granule or a solution. Because of its small molecular weight, its cost is lower than that of a $^{13}C$ octanoic acid. And, because of its water solubility, the rate of adsorption and metabolism is very fast. By incorporating an isotope tracer of a $^{13}C$ glycine, a Tc-99m phytate, a Tc-99m sulfur colloid, a Tc-99m DTPA or a $^{14}C$ glycine with different test meals, a gastric emptying time could be rapidly measured by a $^{13}C$ or $^{14}C$ carbon dioxide breath test or scintigraphy. The test meal obtains several advantages which include an easy preparation, a standardization over the composition and the calorie content, a rapid adsorption and metabolism for a rapid test, a well chemical stability, and a water solubility, comprising a homogeneous dry mix and an isotope tracer provided separately to easily obey the FDA regulations and to get a longer shelf-life. In addition, using fructose as an alternative to sucrose containing formulation is preferable to the diabetes individuals who are often the cases for gastric disorders. In the present invention, the meal components are constituted and cooked on site prior to administering the test. On site preparation of the pre-packaged test meal reduces possibilities on the variability associated with the storage of a pre-cooked meal. This formulation also provides commercial advantages, such as that a dry mix has a longer shelf life and requires no special handling.

BRIEF DESCRIPTION OF THE DRAWINGS

The present invention will be better understood from the following detailed descriptions of the preferred embodiments according to the present invention, taken in conjunction with the accompanying drawings, in which

FIG.1    is a view showing a kit for a gastric emptying measurement according to the present invention;
FIG.2    is a view showing isotope tracer retention in a solid phase of a test meal according to the present invention;
FIG.3    is a view showing the principle of a $^{13}C$ (carbon-13) glycine breath test according to the present invention;
FIG.4    is a view showing an increase in number of a $^{13}C$ atom over a baseline in breath after ingestion according to the present invention;
FIG.5    is a view showing a $^{13}C$ exhaled rate in breath after ingestion according to the present invention;
FIG.6    is a view showing a cumulative $^{13}C$-atom excess over a baseline in breath after ingestion according to the present invention;
FIG.7    is a view showing a $^{13}CO_2$ coefficient variance of the test meal without isotope tracer oral administered according to the present invention;
FIG.8    is a view showing a between-day reproducibility of a solid gastric emptying measurement for the same subject according to the present invention; and

FIG.9    is a view showing a composition of a dry mix according to the present invention.

DETAILED DESCRIPTION OF THE INVENTION

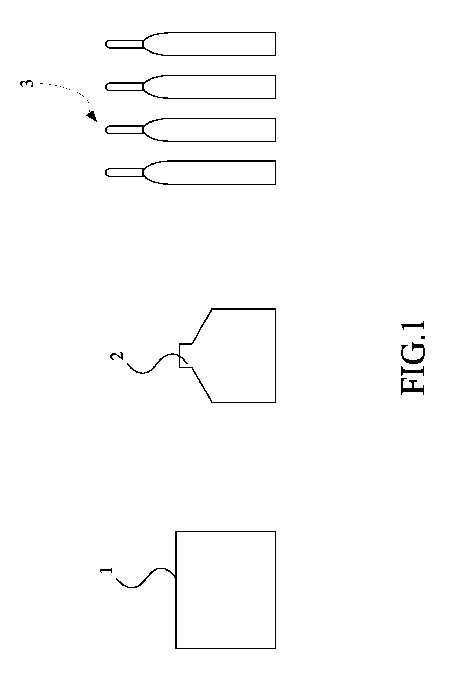

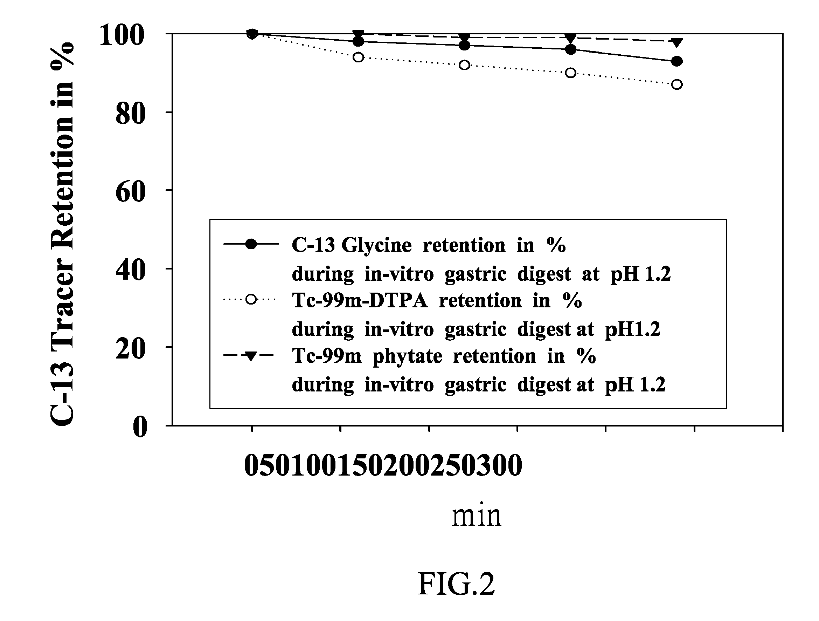

Please refer to FIG.1 and FIG.2, which are views showing a kit for a gastric emptying measurement and isotope tracer retention in a solid phase of a test meal, according to the present invention, where isotope tracer retention percentage is obtained in FIG.2 in vitro gastric digest at pH2.3 to imitate the gastric condition after administering the test meal. As shown in FIG.1, the kit comprises a dry mix 1, which is shipped in a foil, an isotope tracer 2 and a plurality of collecting tubes 3. A standardized recipe is provided to clinicians in the test meal, which has fixed proportions of carbohydrate, protein, and fat. The raw ingredients in the test meal include a lyophilized egg flour. The characteristic is that the isotope tracer 2 is coagulated with albumin of the egg flour into a solid form at more than 75°C, which means the isotope tracer is well incorporated into a food product. More than 90% of the isotope tracer is retained in a mimic gastric fluid at 35~39°C for three hours. The isotope tracer 2 is a $^{13}$C glycine, a $^{14}$C (carbon-14) glycine, a Tc-99m phytate, a Tc-99m sulfur colloid or a Tc-99m DTPA (diethyl-triamine-pentaacetic acid). More than 93% of the $^{13}$C glycine is retained in a mimic gastric fluid at 35~39°C for 4 hours. More than 98% of the Tc-99m phytate is retained in a mimic gastric fluid at 35~39°C for 4 hours. More than 90% of the Tc-99m DTPA is retained in a mimic gastric fluid at 35~39°C for 3 hours. The dry mix 1 comprises egg powder (7.4%), all purpose flour (19.6%), glutinous rice powder (3.9%), whole milk powder (35.7%), levulose (fructose) flour (8%), foaming powder (3%), cream powder (22%), cream fragrance powder (0.2%), and salt (0.2%), having a most preferred calorie of about 299.5±12.5 kcal. The test meal is standardized in a format, for example a format of a muffin or a semisolid soup. Criteria on format selection are concerning convenience, palatability, stable incorporation of the isotope tracer, and capability on being amended on the standardization for the measurement of a gastric emptying. Furthermore, fructose instead of sucrose is preferably offered to patients having diabetes, who are often the cases with gastric disorders. Glycine is a smallest amino acid, so that it has the fastest adsorption and metabolism rate. The present invention is capable of getting a half-gastric emptying time in a shorter period, is cost low, and has a well chemical stability. The chemical stability of the dry mix 1 and stable isotope tracer last more than 2 years.
The present invention costs low and provides a rapid gastric emptying measurement by a carbon breath test or a scintigraphy, comprising the following steps:

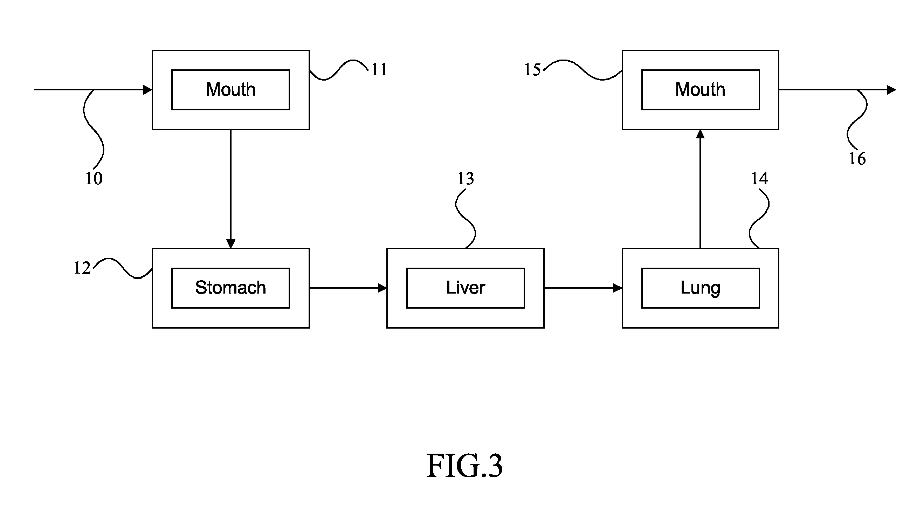

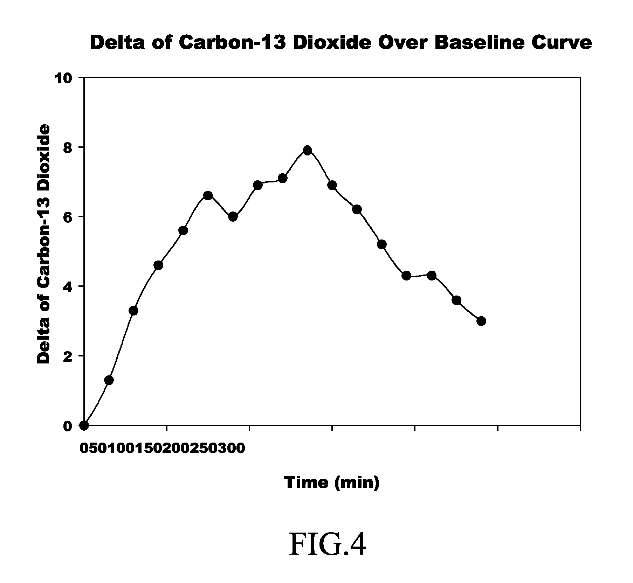

(a) Rapidly constituting a solid test meal comprised with the dry mix1, the isotope tracer 2 and water, by mixing them up, and then cooking the test meal in 9 minutes. If the isotope tracer 2 is a $^{13}$C glycine or a $^{14}$C glycine, then a carbon breath test is used to determine a half-gastric emptying time; if the isotope tracer 2 is a Tc-99m phytate, a Tc-99m-sulfur colloid, or a Tc-99m DTPA, then a scintigraphy is used to determine the half-gastric emptying time. Please refer to FIG.3, which is a view showing the principle of a $^{13}$C glycine breath test according to the present invention. As shown in the figure, the test meal 10 is taken by mouth 11 and is digested in the stomach 12; the $^{13}$C glycine ($NH_2CH_2{}^{13}COOH$) is metabolized in the liver 13 ($H^{13}CO_3^-$ + metabolites); a gas 16 of $^{13}CO_2$ is obtained in lung 14; and, then, the gas 16 is exhaled by the mouth 15 to be collected for a test.
(b) Collecting breath samples from the patients before administering the test meal to the patient.
(c) Oral administering the solid meal with 100mL (milliliter) of water in 10 minutes.
(d) Collecting breath samples each collected from the patient at every 15 minutes during four hours after administering the test meal to the patient.
(e) Measuring a carbon isotope ratio by a mass spectrometer or an infrared spectrometer, if the isotope tracer is a $^{13}$C glycine, to determine the gastric half emptying time of the patient. As shown in FIG.4, the X-axis is sampling time after oral administering the breath meal; and the Y-axis is the increase amount of a $^{13}C/^{12}C$ ratio. So, FIG.4 shows a curve of an increase amount of $^{13}C/^{12}C$ ratio. In a medical examination, the isotope ratio of $^{13}C/^{12}C$ is expressed as $\delta^{13}C$ according to the following equation:

$$\delta^{13}C = \frac{R_s - R_{PDB}}{R_{PDB}} \times 1000(\text{per mil})$$

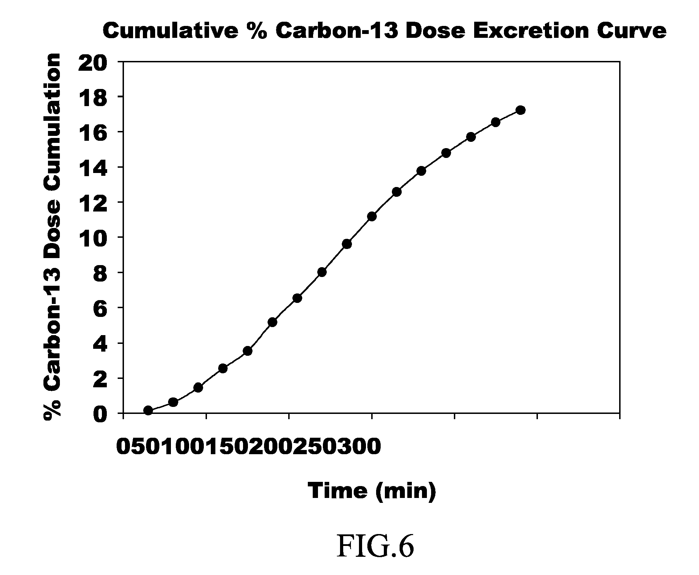

, where the $R_s$ is the isotope ratio of $^{13}C/^{12}C$ in an unknown sample and the PDB is a primary standard whose ratio of $^{13}C/^{12}C$ is 0.0112372.
(f) Converting results obtained from $^{13}CO_2$ breath tests at (e) to %$^{13}$C to be expressed in a percentage of an administered dose of $^{13}$C recovered per hour (i.e. a %$^{13}$C recovery/hr or a %$^{13}$C dose/hr), and to be expressed in a cumulative percentage of the administered dose of $^{13}$C recovered over time (i.e. a %$^{13}$C cumulative dose). The shape of the curve of the %$^{13}$C dose/hr shows the dynamics of the process. It reflects the rate at which the process occurs. The %$^{13}$C cumulative dose, derived numerically from the %$^{13}$C dose/hr data, informs about the global process (as shown in FIG.6).

(g) A non-linear regression analysis is performed on the originally measured data to obtain parameter values of the rate at which stomach empties.

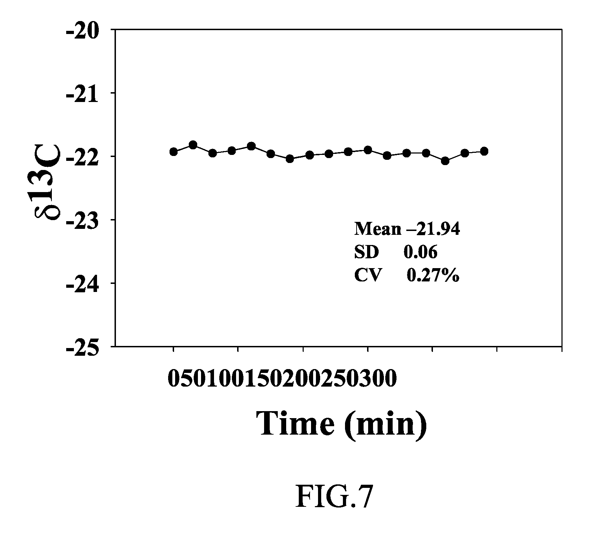

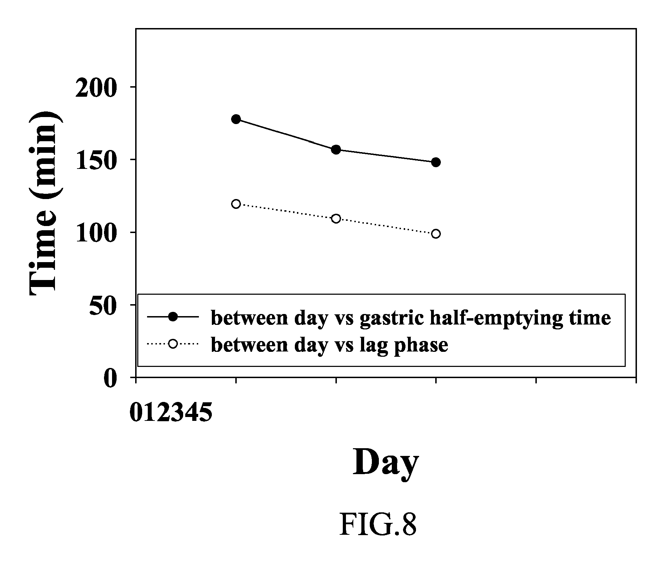

The dry mix 1 uses fructose as an alternative to sucrose containing formulation, where the fructose is preferable to diabetes individuals for long-term follow-up examinations. Besides, the $^{13}C$ abundance of mix 1 ($\delta^{13}C$=-25.5 per mil) is close to the baseline of human breath, which indicates the test meal prepared without isotope tracer did not cause apparent fluctuation of $^{13}C/^{12}C$ ratio in breath test. (CV=0.27% in 4hr after administering to the patients) (see FIG.7). The dry mix 1 and the $^{13}C$ glycine 2 are provided separately for a longer term of storage, which minimize the concerns on stability and FDA regulation. They mimic a true meal and are convenient for palatability, easy preparation, good stability and good precision. The $^{13}C$ glycine 2 combined with the dry mix 1 could be oral administered through stomach to small intestine for a rapid adsorption and a rapid metabolism in liver. The present invention significantly improves current methodologies because it is not limited by the availability of gamma cameras at clinical sites. It is more convenient than a scintigraphic test, because the patient do not have to remain motionless under the expensive instrument. It allows more people to take the test simultaneously. The between-day CV for the same individual is below 10%, which significantly improves the reproducibility of the solid gastric emptying measurement (as shown in FIG.8). In addition, the followings are examples of preferred embodiments in detail:

Example 1 A Test Meal of a Solid Gastric Emptying Measurement

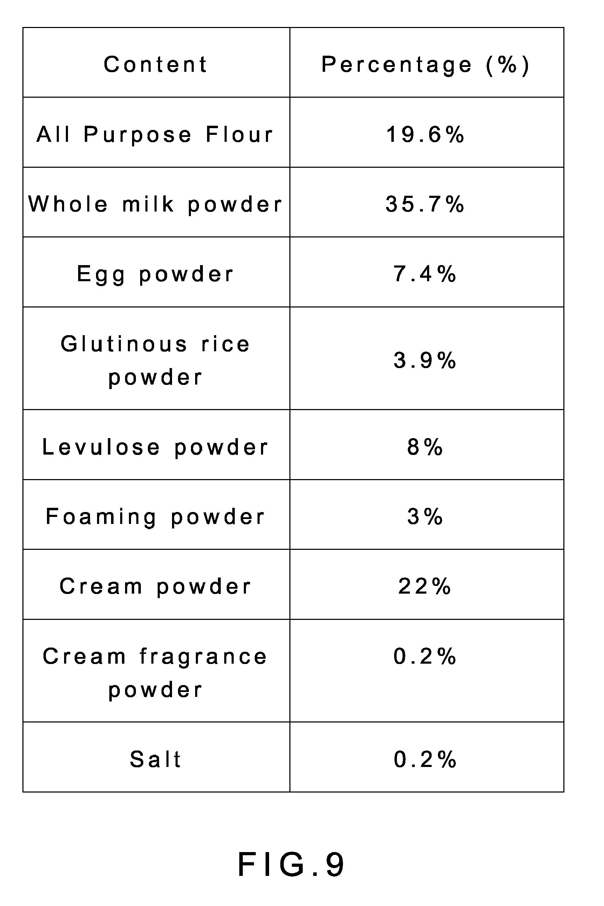

The composition of the dry mix 1 is prepared according to FIG.9. The dry mix 1 is packed in an aluminum foil and is standardized in weight and calorie. The weight is $70\pm3$ grams and the total calorie is $289.5\pm12.5$ kcal. The $^{13}C/^{12}C$ isotope ratio measured with a mass spectrometer is $-25.5\pm0.2$ per mil, which is close to the $^{13}C/^{12}C$ isotope ratio $-24.6\pm1.3$ per mil of the exhaled human breath with a general daily diet and so indicates that the kit's formula and composition are close to a general daily diet (Gut 2002; 51, suppl III, A109.) (Amer J Clin Nutr 1980; 33, 2375.). The shelf-life for the dry mix 1 stored at a room temperature is at least one year and that for a $^{13}C$ glycine is more than 5 years. The $^{13}C$ glycine solid test meal is prepared by putting the dry mix 1 in a container to be mixed with a dissolving $^{13}C$ glycine (50mg (milligram) / 50mL); and then is stirred to be battered and is instantaneously coagulated at more than 75°C for 5 minutes by a waffle iron to produce a test meal in a muffin format. The Tc-99m phytate solid test meal is prepared by putting the flour of the dry mix 1 in a container to be mixed with 1mCi (millicurie) of a Tc-99m phytate and 50mL of water, and then is stirred to be battered and is instantaneously coagulated at more than 75°C for 5 minutes by a waffle iron to produce a test meal in a muffin format. The Tc-99m DTPA solid test meal is prepared by putting the flour of the dry mix 1 in a container to be mixed with 1mCi of a Tc-99m DTPA and 50mL of water; and then is stirred to be battered and is instantaneously coagulated at more than 75°C for 5 minutes by a waffle iron to produce a test meal in a muffin format.

Example 2 An In-Vitro Gastric Simulation

To assess the extent of $^{13}C$ glycine retention in the solid phase of the test meal, a simulated gastric digest is made. A muffin is prepared as described in the section above with 100mg of a $^{13}C$ glycine mixed with the dry mix 1 and water. After chewing the test meal, it is put into a semi-permeable membrane (Spectra/PorMembrane MWCO 3,500, 54mm × 150mm)incubated and shook with a simulated gastric juice (2g (gram) of sodium chloride; 3.2g of pepsin; and, 7mL of HCl in 1000mL, pH1.2) at $37\pm2$°C having different time intervals. 5mL aliquot of the liquid phase were removed at regular 60 min intervals, centrifuged and aliquots of the supernatants removed for C-13 glycine quantification with Liquid Chromatography/Mass Spectrometry. Results are then expressed as a percentage, P%, of the initial amount of the $^{13}C$ glycine added. And, (100-P%) means an incorporation percentage of the $^{13}C$ glycine in the test meal. The results (as shown in FIG.2) show more than 93% of the $^{13}C$ glycine is incorporated in the test meal after 4 hours.

To assess the extent of Tc-99m phytate retention in the solid phase of the test meal, a simulated gastric digest is made. A muffin is prepared as described above with 1mCi of a Tc-99m phytate, which is equivalent to 156.25 $\mu$g (microgram) with a specific activity of 10.06mCi/$\mu$mol ($\mu$mol, micromolar), mixed with the dry mix 1 and water. After chewing the test meal, it is put into a semi-permeable membrane (Spectra/PorMembrane MWCO 3,500, 54mm × 150mm) incubated and shook with a simulated gastric fluid (2g of sodium chloride, 3.2g of pepsin and 7mL of HCl in 1000mL, pH1.2) at $37\pm2$°C having different time intervals. 5mL aliquot of the liquid phase were removed at regular 60 min intervals, centrifuged and aliquots of the supernatants removed for liquid scintillation counting. Results are then expressed as a percentage, P%, of the initial amount of a radioactivity added. And, (100-P%) means an incorporation percentage of the Tc-99m phytate in the test meal. The results (as shown in FIG.2) show more than 98% of the Tc-99m phytate is incorporated in the test meal after 4 hours.

To assess the extent of Tc-99m DTPA retention in the solid phase of the test meal, a simulated gastric digest is made. A muffin is prepared as described above with 1mCi of a Tc-99m DTPA, which is equivalent to 110.22$\mu$g with a specific activity of 4.54 mCi/$\mu$mol, mixed with the dry mix 1 and water. After chewing the test meal, it is put into a semipermeable membrane (Spectra/ PorMembrane MWCO 3,500, 54mm $\times$ 150mm) incubated and shook with a simulated gastric fluid (2g of sodium chloride, 3.2g of pepsin and 7mL of HCl in 1000mL, pH1.2) at 37$\pm$2°C having different time intervals. 5mL aliquot of the liquid phase were removed at regular 60 min intervals, centrifuged and aliquots of the supernatants removed for liquid scintillation counting. Results are then expressed as a percentage, P%, of the initial amount of a radioactivity added. And, (100-P%) means an incorporation percentage of the Tc-99m DTPA in the test meal. The results (as shown in FIG.2) show more than 87% of Tc-99m DTPA is incorporated in the test meal after 4hours.

Example 3 The Stability and Suitability of a Test Meal for the Solid Gastric Emptying Measurement

To perform a gastric emptying test, a baseline sample of breath is collected using a septum capped glass tube in the morning after an overnight fast; and then is analyzed to obtain a baseline $\delta^{13}$C level. The blank solid test meal is prepared by putting the flour of the dry mix 1 along with water in a container; and then is stirred to be battered and is instantaneously coagulated at more than 75°C for 5 minutes by a waffle iron to produce a blank test meal in a muffin format. The patient then administered the blank test meal along with 100mL water within 10 minutes. The breath samples are collected with a 15-minute interval for 4 hours and analyzed using an isotope ratio mass spectrometer and are plotted into FIG.7. The X-axis of FIG.7 is a sampling time and the Y-axis of FIG.7 is the $^{13}$C/$^{12}$C isotope ratio ($\delta^{13}$C). The curve shows the variation of the breath samples is only 0.27%. It means the fluctuation of the baseline of the breath tests for the dry mix 1 of the test meal is very low and stable; and do not affect the results of the breath tests. The $^{13}$C abundance of dry mix 1 was determined to be -25.5$\pm$0.2 per mil with an isotope ratio mass spectrometer, which closely approximates the $^{13}$C abundance of fasting breath $CO_2$ from the patients. Besides, the low baseline fluctuation indicates it will not alter the $CO_2$ abundance in breath test (Am. J. Clin. Nutr. 33:2375~2385, 1980).

Example 4 The Quantification of a Half-Emptying Time and Lag Phase Time

A test meal is prepared by putting the dry mix 1 along with a dissolving $^{13}$C glycine (50mg/50mL) in a container; and then is stirred to be battered and is instantaneously coagulated at more than 75°C for 5 minutes by a waffle iron to produce a test meal in a muffin format. The test meal is administered after an overnight fast along with 100mL of water within 10 minutes. The first one of the breath samples is collected before the test meal is administered so that a baseline is obtained. And the rest of the breath samples are collected with an 15-minute intervals during 4 hours after the test meal is administered. A measurement can be conveniently done sing an isotope ratio mass spectrometer. The results obtained are then expressed in a $\delta^{13}$C value ($^{13}$C/$^{12}$C). FIG.6 shows a cumulative %$^{13}$C dose excretion curve of the breath tests, which resembles the reversed retention curve. The $^{13}CO_2$ excretion parameters are derived from the chi-square distribution in statistics and the equation is expressed as follows.

$$CD = m(1-e^{-kt})^{\beta} \quad ,$$

where CD is the cumulative percentage of the administered dose; t is the time; and, m is the total cumulative percentage of the dose recovered.

A non-linear regression analysis is performed on the originally measured data to obtain values of the m, k, and $\beta$ for each individual breath test.

A. Half emptying time:

The half emptying time is calculated by making CD equal to m/2 in the CD equation:

$$t^{\frac{1}{2}} = (-\frac{1}{k})\ln(1-2^{-\frac{1}{\beta}})$$

B. Lag phase:

The lag phase for the breath test has been defined, which is expressed as follows:

$$t_{lag} = 1/k \ln \beta$$

[Example 5] An Example of a Test Calculation

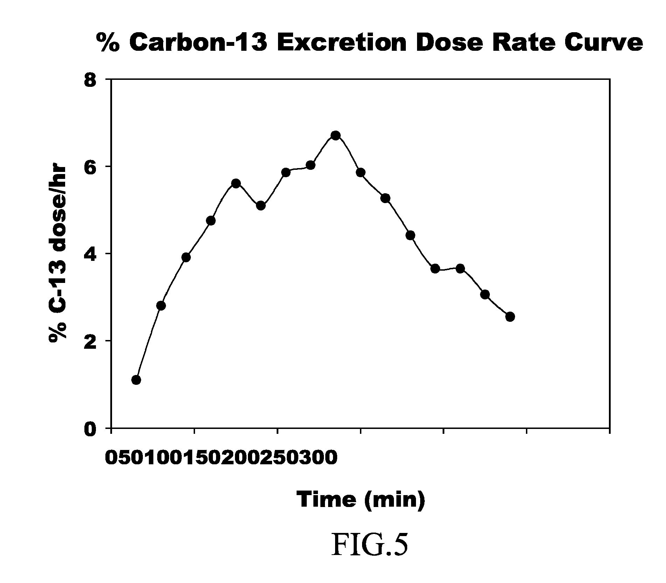

(1)    Patient: X
(2)    Weight (W): 60kg
(3)    Height (H): 164cm
(4)    Body Surface Area, BSA:
    $BSA = (W^{0.5378} \times H^{0.3964}) \times 0.024265$
    = 1.6566 (unit : $m^2$ , meter square)
    $BSA = (W^{0.5378} \times H^{0.3964}) \times 0.024265$, which is calculated according to the formula ofHaycock et al. (J. Pediatr., 93, 62-66, 1978.)
    W means weight (in kg, kilogram)
    H means height (in cm, centimeter)
(5)    $CO_2$ production
    = 300 (mmol/$m^2 \cdot$ hr) $\times$ BSA ($m^2$)
    = 497 (mmol/hr)
    (mmol, millimolar; m, meter)
(6)    substrate ($^{13}$C-glycine) mg administered = 50mg
(7)    %$^{13}$C-substrate=99 atom%
(8)    molecular weight of $^{13}$C-glycine
    = 76.06 mg/mmol
(9)    n=1 (only the carboxyl group of glycine is $^{13}$C labeled)
(10)    measuring $^{13}$C/$^{12}$C of breath samples using an isotope ratio mass spectrometer ($\delta^{13}$C, per mil)
(11)    obtaining a $\Delta\delta^{13}$C of breath samples at each sampling time by subtracting $\delta^{13}$C collected at time zero from $\delta13$C at each sampling time. Results of $\Delta\delta^{13}$C are shown in FIG.4.
(12)    The $\Delta\delta^{13}$C value obtained by the mass spectrometric analysis is converted to %$^{13}$C; and results on $^{13}CO_2$ breath tests are expressed in percentage of the administered dose of $^{13}$C recovered per hour (i.e. %$^{13}$C recovery/hr or %$^{13}$C dose/hr). FIG.5 represents a $^{13}CO_2$ excretion (in %$^{13}$C dose/hr) in a course of time. The shape of the %$^{13}$C dose/hr curve shows the dynamics of the process. It reflects the rate at which the process occurs (delayed, accelerated, with or without a lag phase). The %$^{13}$C dose/hr is calculated by the following equation Eq.1:

$$\%^{13}C \text{ dose/hr} = \frac{\text{mmol } ^{13}C \text{ excess in breath (a)}}{\text{mmol } ^{13}C \text{ excess administered (b)}} \times 100 \text{ -------------------------------Eq.1}$$

The definition of mmol $^{13}$C excess in breath (a) and mmol $^{13}$C excess administered (b) were shown below:

(a) mmol $^{13}$C excess in breath

$$= \left( \frac{\%^{13}C_t - \%^{13}C_{t_0}}{100} \right) \times CO_2 \text{ production}$$

$$\%13C_t = \frac{\left( \frac{\delta t}{1000} + 1 \right) \times 0.0112372}{\left( \left( \frac{\delta t}{1000} + 1 \right) \times 0.0112372 \right) + 1}$$

0.0112372 is the ratio of $^{13}C/^{12}C$ of PDB

$\%^{13}C_t$ and $\%^{13}C_{t0}$ : the concentration of $^{13}C$ at time t and to (i.e. time zero)

$\delta t = \delta^{13}C$ value at time t

$(\%^{13}C_t - \%^{13}C_{t0})$ is also called "$^{13}C$ atom percent excess"

(b) mmol $^{13}C$ excess administered

$$= \left( \frac{\%^{13}C_{substr.} - \%^{13}C_{t_0}}{100} \right) \times \frac{m}{M} \times n$$

$\%^{13}C_{substr} = \%^{13}C$ present in substrate

M = molar mass of substrate

m = amount of substrate

n = number of atoms, $^{13}C$-labelled

(13) The $\%^{13}C$ cumulative dose is derived numerically from the $\%^{13}C$ dose/hr data and is calculated from the following Eq.2, which informs about the global process. The $^{13}C$ cumulative excretion is shown in FIG.6.

$$\%^{13}C_{cumul.\ dose\ t_i+1}$$

$$= \%^{13}C_{cumul.\ dose\ t_i} +$$

$$\left( \frac{\%^{13}C_{dose\ t_i} + \%^{13}C_{dose\ t_{i+1}}}{2} \right) \times \frac{1}{n} \text{-----------------------Eq.2}$$

n = number of samples per hour

n = 4, if a breath sample is taken every 15 minutes

$t_i$ = time i

The numerical calculation is given as follows in detail:

| Sampling time (min) | $\delta^{13}C$ (per mil) | $\Delta\delta^{13}C$ (per mil) | $\%^{13}C$ (dose/hr) | $\%^{13}C_{cumul.\ dose}$ |
|---|---|---|---|---|
| 0 | -21.6 | 0 | ---- | ------ |
| 15 | -20.3 | 1.3 | 1.10377 | 0.13797 |
| 30 | -18.3 | 3.3 | 2.80151 | 0.61787 |
| 45 | -17.0 | 4.6 | 3.90500 | 1.45618 |
| 60 | -16.0 | 5.6 | 4.75382 | 2.53853 |
| 75 | -15.0 | 6.6 | 5.60262 | 3.53309 |
| 90 | -15.6 | 6.0 | 5.09334 | 5.17009 |
| 105 | -14.7 | 6.9 | 5.85726 | 6.53892 |
| 120 | -14.5 | 7.1 | 6.02701 | 8.02445 |
| 135 | -13.7 | 7.9 | 6.70603 | 9.61608 |

(continued)

| Sampling time (min) | $\delta^{13}C$ (per mil) | $\Delta\delta^{13}C$ (per mil) | $\%^{13}C$ (dose/hr) | $\%^{13}C_{cumul.\ dose}$ |
|---|---|---|---|---|
| 150 | -14.7 | 6.9 | 5.85726 | 11.18649 |
| 165 | -15.4 | 6.2 | 5.26310 | 12.57654 |
| 180 | -16.4 | 5.2 | 4.41430 | 13.78622 |
| 195 | -17.3 | 4.3 | 3.65035 | 14.79430 |
| 210 | -17.3 | 4.3 | 3.65035 | 15.70689 |
| 225 | -18.0 | 3.6 | 3.05617 | 16.54521 |
| 240 | -18.6 | 3.0 | 2.54685 | 17.24559 |

(14)  Mathematically analyzing the $^{13}CO_2$ excretion curves with a Sigmaplot software. The cumulative $\%^{13}C$ dose excretion curve of the breath test (as shown in FIG.6) is described as an equation: $CD = m(1-e^{-kt})^\beta$; and, m=22.844, k=0.0101 and $\beta$=2.8256 arc obtained. All of these parameters were determined by a non-linear regression analysis. The half emptying time is calculated by making CD equal to m/2 in the CD equation and being defined by

$$t^{\frac{1}{2}} = (-\frac{1}{k})\ln(1-2^{-\frac{1}{\beta}}) \text{-----------------------------------------------------} Eq.3$$

The half-emptying time is equal to 151.0 minutes, which is calculated by entering these parameters of k and $\beta$ into the equation of Eq.3. The lag phase for the breath test is expressed as $t_{lag}$ = 1/k ln $\beta$ (Eq.4). The emptying delayed time is equal to 102 minutes, which is calculated by entering these parameters of k and $\beta$ into the equation of Eq.4.

Example 6 A Between-day Reproducibility of the Solid Gastric Emptying Measurement for the Same Subject

The reproducibility of the solid gastric emptying measurement is investigated by thirty-five volunteers within a one-week period using a $^{13}C$ breath test. The dry mix 1, water and a $^{13}C$-glycine are mixed thoroughly. The mix is cooked exactly as directed, and is cooled to a room temperature. The patients arrive at the clinician's facility after an overnight fast and a baseline sample of $CO_2$ is collected from the patients. The test meal is administered with 100mL of water. The patients remain within a certain area throughout the test. Samples are continuously collected with a 15-minute interval for 4 hours. The appearance of label is measured appropriately. The $^{13}CO_2$ excretion curves are mathematically analyzed using a non-linear regression method to get the half-emptying time ($t_{1/2}$) and the lag phase time ($t_{1ag}$, emptying delayed time). The between-day coefficient variance is below 10%, either for $t_{1ag}$ or $t_{1/2}$ (as shown in FIG.8).

To sum up, the present invention relates to a $^{13}C$-glycine kit for solid or semisolid gastric emptying measurement. The test meal is made in a muffin form by a quick coagulation of albumin with the other constituents in the dry mix 1 together with the $^{13}C$-glycine cooked at more than 75°C. The incorporation of the isotope tracer 2 mixed into the muffin and the characteristics of the $^{13}C$- glycine are the major causes for a good precision and stability during the gastric emptying measurement. The measurement is finished soon even including the preparation of the test meal. Besides, the test meal is easy for a quick preparation. The advantages also include the low cost of the $^{13}C$- glycine, a long shelf-life of the $^{13}C$- glycine, and the palatability provided. The isotope tracer and the dry mix are provided separately, which makes it easy for the quality and quantity control and is easy to obey the FDA regulations. Furthermore, fructose is chosen as an alternative to sucrose containing formulation is preferred for the diabetes which is often the case for a gastric disorder. So, the present invention allows an accurate standardization and a more convenient measurement for gastric emptying results.

The preferred embodiments herein disclosed are not intended to unnecessarily limit the scope of the invention. Therefore, simple modifications or variations belonging to the equivalent of the scope of the claims and the instructions disclosed herein for a patent are all within the scope of the present invention.

**Claims**

1. A kit for a gastric emptying measurement, comprising:

   a dry mix having an egg flour, said egg flour obtained from egg having an albumin, said egg flour lyophilized and powdered; and
   an isotope tracer,
   wherein said isotope tracer is selected from a group consisting of a carbon-13 ($^{13}$C) glycine, a carbon-14 ($^{14}$C) glycine, a Tc-99m (metastable Technetium- 99) phytate, a Tc-99m sulfur colloid, and a Tc-99m DTPA (diethyl - triamine - pentaacetic acid);
   wherein a test meal is obtained within 9 minutes from said dry mix mixing with said isotope tracer; and
   wherein said albumin of said dry mix is coagulated at a temperature more than 75°C.

2. The kit according to claim 1,
   wherein said dry mix comprises said egg flour, a plain flour, a wafer flour, a whole-fat milk powder, a fructose flour, a soda flour, a butter flour, a butter herb, and salt; and
   wherein said dry mix comprises no glucose and no sucrose.

3. The kit according to claim 1 or 2, wherein said test meal is obtained in a form selected from a group consisting of a solid form and a semisolid form.

4. The kit according to one of claims 1 to 3, wherein total calories of said test meal is between 302kcal (kilocalorie) and 277kcal.

5. The kit according to one of claims 1 to 4, wherein said isotope tracer is obtained in a form selected from a group consisting of a crystal, a capsule, a tablet, a granule, or a solution.

6. The kit according to one of claims 1 to 5,
   wherein said test meal contains said isotope tracer;
   wherein said isotope tracer is selected from a group consisting of a $^{13}$C or a $^{14}$C or a Tc-99m; and
   wherein an isotope tracer retention in a solid phase of said test meal is more than 90% at a temperature between 35°C and 39°C for more than 3 hours under a simulated gastric fluid.

7. The kit according to one of claims 1 to 6, wherein the kit has a testing procedure comprising steps of:

   within said 9 minutes, rapidly constituting said test meal into said solid form by mixing said dry mix and said isotope tracer with water to be filled into a waffle iron to be cooked at a temperature more than 75°C;
   by using a collecting tube and a straw, collecting a first sample of air from a patient having an overnight fast as a baseline;
   administering said test meal with 100mL (milliliter) of water by said patient within 10 minutes and starting counting a half emptying time right after finishing administering said test meal; and
   measuring the appearance of label of said isotope tracer by a method coordinated with an instrument for figuring out said half emptying time,
   wherein said first sample comprises two collecting tubes each containing air from one of two sequential breathing-out of said patient; and
   wherein said isotope tracer is a $^{13}$C-glycine, said instrument is selected from a group consisting of a mass spectrometer or an infrared spectrometer, and said kit is a kit for a carbon isotope breath test.

8. The testing procedure according to claim 7, wherein said isotope tracer is a $^{14}$C-glycine, and said instrument is a β scintillation counter.

9. The testing procedure according to claim 7, wherein said isotope tracer is selected from a group consisting of a Tc-99m phytate, a Tc-99m sulfur colloid, and a Tc-99m DTPA, said instrument is a nuclear scintigraphic instrument, and said kit is a kit for a scintigraphy analysis.

10. The testing procedure according to claim 7 or 8, wherein a coefficient variance of said half emptying time for said test meal is below 10%.

11. The testing procedure according to one of claims 7 to 10, wherein the $^{13}C$ abundance of said test meal is between -25.3 and -25.7 per mil.

12. The testing procedure according to one of claims 7 to 11, wherein said method comprises steps of:

obtaining samples of air from said patient each collected every 15 minutes during 4 hours after starting counting said half emptying time; and

together with said first sample collected in step (2), analyzing said samples collected in step (a) to figure out: [I] increases in a $^{13}C/^{12}C$ isotope ratio, [II] increases in a recovery ratio of $^{13}C$, [III] increases in a cumulative dose of $^{13}C$, [IV] a half emptying time, and [V] a lag phase time.

13. The testing procedure according to claim 12, wherein said isotope tracer is $^{14}C$ and, in steps (b), said samples are analyzed to figure out: [I] increases in a β-scintillation counting, [II] increases in a recovery ratio of $^{14}C$, [III] increases in a cumulative dose of $^{14}C$, [IV] a half emptying time, and [V] an emptying delayed time.

14. The testing procedure according to claim 7, wherein a $\delta^{13}C$ variation of said blank test meal administered is around 0.27% for over a 4-hr period.

15. A kit for a gastric emptying measurement according to claims 1, wherein said egg flour is obtained from whole egg, and wherein said tracer is a $^{13}C$-glycine.

16. The kit according to claim 15, wherein said dry mix comprises said egg flour, a plain flour, a wafer flour, a whole-fat milk powder, a fructose flour, a soda flour, a butter flour, a butter herb, and salt; wherein said test meal is obtained in a form selected from a group consisting of a solid form -or a semisolid form; wherein total calories of said test meal is between 302kcal and 277kcal; wherein said isotope tracer is obtained in a form selected from a group consisting of a crystal, a capsule, a tablet, a granule, and a solution; and wherein a $^{13}C$-glycine retention in a solid phase of said test meal at a temperature between 35°C and 39°C for more than 4 hours under a simulated gastric fluid is more than 93%.

17. The kit according to claim 15 or 16, wherein the kit has a testing procedure comprising steps of:

Within said 9 minutes, rapidly constituting said test meal into said solid form by mixing said dry mix and said isotope tracer with water to be filled into a waffle iron to be cooked at a temperature more than 75°C;

By using a collecting tube and a straw, collecting a first sample of air from a patient having an overnight fast as a baseline;

Administering said test meal with 100mL of water by said patient within 10 minutes and starting counting a half emptying time right after finishing administering said test meal;

Obtaining breath samples from said patient each collected every 15 minutes during 4 hours after starting counting said half emptying time, said sample comprising two collecting tubes each containing air from one of two sequential breathing-out of said patient respectively; and

Coordinated with an instrument, analyzing said samples collected in step (4) together with said first sample collected in step (2) to figure out: [I] increases in a $^{13}C/^{12}C$ isotope ratio, [II] increases in a recovery ratio of $^{13}C$, [III] increases in a cumulative dose of $^{13}C$, [IV] a half emptying time, and [V] an lag phase time.

18. The testing procedure according to claim 17, wherein a baseline $\delta^{13}C$ variation is around 0.27%; and wherein said instrument is selected from a group consisting of a mass spectrometer and an infrared spectrometer.

19. The testing procedure according to claim 17, wherein said isotope tracer is a $^{14}C$-glycine; wherein said instrument is a β scintillation counter; and wherein, in step (5), the followings are figured out: [I] increases in a β-scintillation count, [II] increases in a recovery ratio of $^{14}C$, [III] increases in a cumulative dose of $^{14}C$, [IV] a half emptying time, and [V] an emptying delayed time; and wherein a $^{14}C$-glycine retention in a solid phase of said test meal at a temperature between 35°C and 39°C for more than 4 hours under a simulated gastric juice is more than 90%.

FIG.1

FIG.2

Mouth 15

16

Mouth 11

Lung 14

10

Stomach 12

Liver 13

FIG.3

FIG.4

% Carbon-13 Excretion Dose Rate Curve

FIG.5

EP 1 707 219 A2

**Cumulative % Carbon-13 Dose Excretion Curve**

FIG.6

FIG.7

EP 1 707 219 A2

FIG.8

| Content | Percentage (%) |
| --- | --- |
| All Purpose Flour | 19.6% |
| Whole milk powder | 35.7% |
| Egg powder | 7.4% |
| Glutinous rice powder | 3.9% |
| Levulose powder | 8% |
| Foaming powder | 3% |
| Cream powder | 22% |
| Cream fragrance powder | 0.2% |
| Salt | 0.2% |

FIG.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02062399 A1, Meiler
- WO 0172342 A1, Ghoos
- US 6548043 B1, Wagner

### Non-patent literature cited in the description

- **GHOOS et al.** Measurement of Gastric Emptying Rate of Solids by Means of a Carbon-Labeled Octanoic Acid Breath Test. *Gastroenterology,* 1993, vol. 104, 1640-1647
- **MAES et al.** Combined Carbon-13-Glycine/Carbon-14-Octanoic Acid Breath Test to Monitor Gastric Emptying Rates of Liquids and Solids. *J Nucl Med,* 1994, vol. 35, 824-831
- *Amer J Clin Nutr,* 1980, vol. 33, 2375
- *Am. J. Clin. Nutr.,* 1980, vol. 33, 2375-2385
- **HAYCOCK et al.** *J. Pediatr.,* 1978, vol. 93, 62-66